# EUROPEAN PATENT SPECIFICATION

(11) **EP 2 379 003 B1**
(45) Date of publication and mention of the grant of the patent: **21.10.2015**
(21) Application number: 09760105.8
(22) Date of filing: 19.11.2009
(51) Int. Cl.: A61F 2/00

(54) **Planar implant for sealing liquid and/or air leaks in the human and/or animal body, and method for its production**
Planares Implantat zur Abdichtung von Flüssigkeits- und/oder Luftlecks im menschlichen und/oder tierischen Körper und Herstellungsverfahren
Implant plan pour étanchéifier des fuites de liquide et/ou d'air dans le corps humain et/ou animal, et son procédé de fabrication

(30) Priority: 20.11.2008 DE 102008059245
(43) Date of publication of application: 26.10.2011
(73) Proprietor: Aesculap AG, 78532 Tuttlingen (DE)
(72) Inventor: BLENDER, Bernd, 88367 Hohentengen (DE); WEGMANN, Jürgen, 78333 Stockach (DE); ODERMATT, Erich, CH-8200 Schaffhausen (CH)
(74) Representative: Patentanwälte Ruff, Wilhelm, Beier, Dauster & Partner mbB
(86) International application number: PCT/EP2009/008230
(87) International publication number: WO 2010/057632

(56) References cited:
- WO-A-03/099160
- WO-A-2008/002815
- DE-A1- 2 625 289
- US-A1- 2007 161 109
- US-A1- 2008 167 729
- US-B1- 6 398 814

## Description

The present invention relates to a planar implant, which is suitable for sealing liquid and/or air leaks in the human and/or animal body, and to a method for production of the implant.

Haemostatics are generally used to stop bleeding. An example of a possible area of use is that of internal haemostasis, for example of organ haemorrhages. A further area of use concerns the closure of leaks from the lungs. The haemostatics are generally fleece-like, i.e. web or fabric type, structures that can be made from polysaccharides, for example oxidized cellulose, or proteins, in particular collagen.

Because of their biocompatible and absorbent properties, collagen fleeces are particularly suitable for use as haemostatics. Collagen fleeces are generally produced from freeze-dried, homogeneous collagen suspensions. A precondition for efficient haemostasis by collagen fleeces is a certain degree of absorbency for body fluids, particularly blood. The absorbency of collagen fleeces is dependent on a large number of factors. Of particular importance in this connection is the porosity, the surface area, the fibre density and the thickness of the collagen fleeces.

Permanent optimization of the factors mentioned in the preceding paragraph often leads to stiff fleece structures. For example, thick fleece structures with suitable porosity and a suitable surface area provide good absorbency in respect of body fluids. However, starting from a certain fleece thickness, the flexural strength and brittleness of the fleeces increase, as a result of which they can be modelled only with difficulty to curved or arcuate wound areas. By contrast, if the fleece structures have a small thickness, they generally have a flexibility sufficient for their application, but the small thickness means that they often have an unsatisfactory absorption capacity in respect of body fluids. An example of a two-layer or multi-layer collagen product is described in US 2007/0161109 A1. A disadvantage thereof is in principle the two-layer or multi-layer structure of the product, as this can lead to an increased flexural strength and, if appropriate, brittleness of the collagen product.

The haemostatics commercially available at present and based on collagen fleece generally have a thickness of < 4 mm. To improve handling, in particular to make modelling to the wound surfaces easier, the collagen fleeces are partially moistened before being applied. However, some of the original absorbency is lost as a result of the preliminary moistening. This in turn leads to reduced haemostatic performance of the moistened fleeces. In order to obtain satisfactory haemostasis, collagen fleeces are often used with proteins from the coagulation cascade, in particular thrombin and fibrinogen. However, the recovery of such proteins is generally very difficult, which greatly increases the costs of the production method. Moreover, in principle, the use of such proteins also increases the risk of infection.

WO 2008/002815 A2 discloses an anti-adhesion sheet with pending zones in form of recesses. US 6,398,814 B1 discloses a multi-layer two-dimensional composite device which is made of synthetic polymers or copolymers such as copolymers of ε-caprolactone or of L-lactic acid and D, L-lactic acid. WO 03/099160 A1 relates to a medical implant having a knobbed film. US 2008/0167729 A1 discloses an implantable device for reinforcing tissue surrounding a surgically created stoma in a patient, wherein a sizing pattern is applied to the surface of the device.

The object of the present invention is therefore to make available an implant which avoids the problems of conventional haemostatics and which in particular has sufficient flexibility for modelling it onto tissue surfaces, especially onto arcuate and/or curved tissue surfaces, without adversely affecting the absorbency and in particular the haemostatic properties of the implant.

According to the invention, this object is achieved by a planar implant having the features of independent Claim 1. Preferred embodiments of the implant form the subject matter of dependent Claims 2 to 14. Moreover, the invention also relates to a method for the production of the implant, having the features of Claim 15. A preferred embodiment of the method forms the subject-matter of claim 16.

The implant according to the invention is a planar implant, for sealing liquid and/or air leaks in the human and/or animal body, wherein the implant has a surface with predetermined bending locations in the form of depressions.

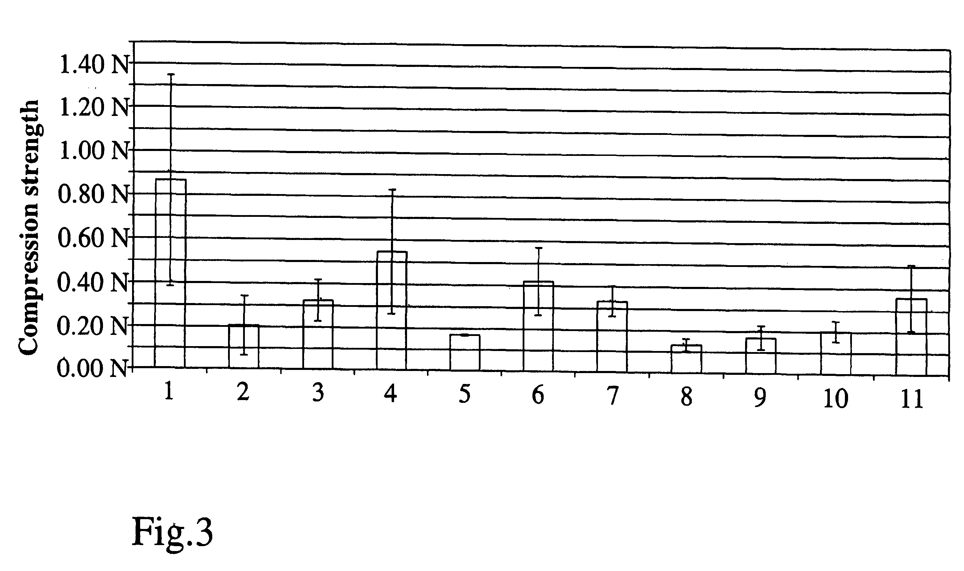

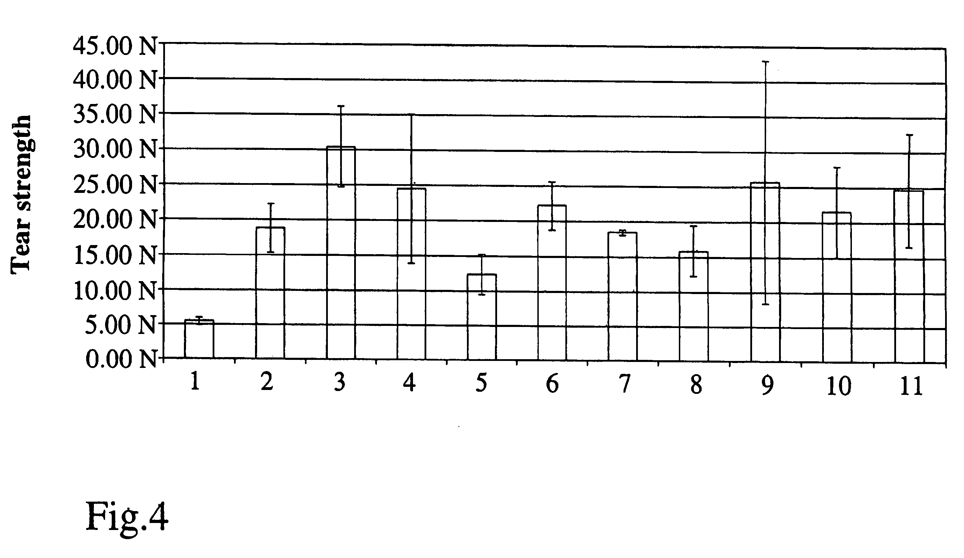

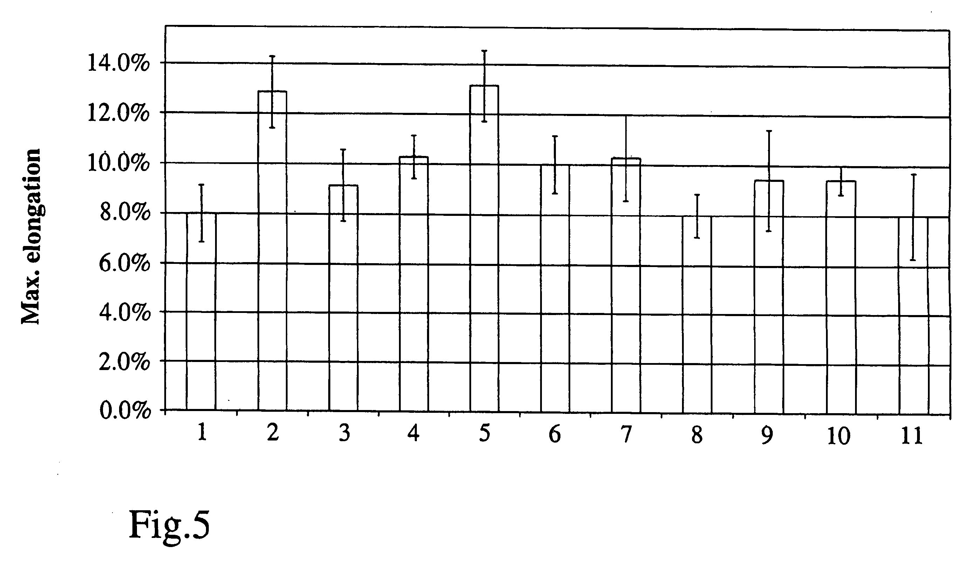

By means of the predetermined bending locations or predetermined buckling locations provided according to the invention in the form of depressions on the implant surface, the implant is particularly advantageously flexible, in particular bendable. By virtue of the fact that the implant surface drops down to the depressions at an angle, no tensile stress acts on the implant during bending, and the risk of the implant breaking is thereby greatly reduced. Moreover, preliminary treatment in the form of moistening is no longer absolutely necessary before application, for example before application to a wound surface. Instead, the implant according to the invention can be modelled onto a curved or arcuate tissue surface without being moistened, and without breaks occurring in the implant. It is thus possible to avoid the loss of absorbency caused by preliminary treatment and to ensure that the haemostatic properties in particular of the implant are not affected undesirably. A further advantage is that less moistening of the implant is sufficient to be able to model the implant onto the site of application. Moreover, it has surprisingly been found that the implant according to the invention is not only much more flexible than conventional haemostatics (in this connection see the results shown in graph form in Figure 3) but at the same time also has increased stability (in this connection see the results shown in graph form in Figures 4 and 5).

The plural "depressions" is to be understood within the meaning of the present invention as one, two, three or more depressions, in particular a multiplicity of depressions. In general, there are several depressions, in particular a multiplicity of depressions, formed on the implant surface. Normally, the depressions entail the arrangement of elevations on the surface of the implant. The nature of the elevations, for example their size, form, shape and/or orientation of the implant's surface, is preferably dependent from the nature of the depressions, for example their size, form, shape, and/or orientation of the implant's surface.

In a preferred embodiment, the depressions are elongate. Elongate depressions are to be understood within the meaning of the present invention as depressions that have a length to width ratio of > 1 (length > width). In a particularly preferred embodiment, the depressions are linear. The depressions are preferably groove-shaped or channel-shaped.

In another suitable embodiment, the depressions are continuous, preferably as seen in the longitudinal direction of the implant. In other words, the depressions on the implant surface can be uninterrupted or have no interruptions. In an alternative embodiment, the depressions are interrupted or have interruptions. In particular, the depressions can be formed in the manner of perforation lines on the implant surface. For example, the depressions can be formed one behind another, i.e. arranged in rows, on the implant surface.

In another embodiment, the depressions are point-shaped and/or trough-shaped, preferably point-shaped. Point-shaped depressions within the meaning of the present invention are to be understood as depressions that have a length to width ratio of 1 (length = width).

According to the invention, provision can also be made for the implant surface to have differently shaped depressions. For example, the implant surface can have elongate and also trough-shaped and/or point-shaped depressions. For further features and details in this connection, reference is made to the preceding description and to the description below.

The implant, in the area of the depressions, is compacted, in particular pressed, preferably embossed. Compacted depressions particularly advantageously represent areas of increased material strength of the implant that are suitable, for example, for securing, in particular suturing, the implant to body structures, generally to tissues. The depressions are preferably pressed depressions, in particular punched or embossed depressions.

The depressions can be formed in the longitudinal and/or transverse direction of the implant. The depressions are preferably formed in the longitudinal and transverse directions of the implant. Moreover, the depressions can be formed in the oblique direction, in particular the diagonal direction, of the implant.

In a preferred embodiment, the depressions are formed at least partially, in particular completely, parallel to one another on the surface of the implant. For example, the depressions can extend parallel to the longitudinal direction of the implant. This has the advantage that the depressions can serve as a guiding line for a user, generally a surgeon, when cutting the implant to a suitable format for application.

According to the invention, provision can also be made for the depressions to be offset relative to one another on the surface of the implant. In other embodiments, the depressions are formed on the surface of the implant at right angles to one another or at angles of other than 90 degrees to one another.

The depressions are formed on the surface of the implant in a regular arrangement in the form of a pattern. A regular arrangement of depressions usually necessarily entails a similarly regular arrangement of elevations on the implant surface, which elevations are surrounded by the depressions. The depressions are formed on the implant surface in a lattice or net shape. According to the invention, the depressions can be formed on the implant surface in a grid size of between 0.5 and 3 cm, preferably of between 1 and 1.5 cm.

The depressions are formed on the surface of the implant in the form of a pattern. The patterns can in principle include all conceivable geometries. According to the invention, it is particularly preferable if the depressions are formed on the implant surface in the form of a polygonal pattern, in particular in the form of a pattern of triangles, diamonds, lozenges, squares, rectangles, pentagons or hexagons.

In another embodiment, the depressions are formed on the surface of the implant in an arcuate configuration, in particular in a curving and/or meandering shape. In principle, the depressions can also be formed on the surface of the implant in the form of graphic symbols, logos or motifs.

The depressions are generally formed on at least one side face, preferably on at least one contact face, of the implant. According to the invention, provision can also be made for the depressions to be formed on two side faces, in particular on two opposite side faces, of the implant. In particular, the depressions can be formed to different extents on two opposite side faces of the implant.

In suitable embodiments, the depressions have a width of between 0.1 and 5 mm, in particular of between 0.5 and 1.5 mm. In the area of the depressions, the implant preferably has a thickness of between 1 and 90%, preferably of between 20 and 80%, particularly preferably of between 60 and 80%, relative to a cross section of the implant without depressions.

In the dry state, the implant preferably has a tear strength of between 10 and 75 N, in particular of between 15 and 35 N, in its longitudinal direction. In its longitudinal direction, the implant, in the dry state, preferably has an elongation at break of between 5 and 80%, in particular of between 15 and 75%, preferably of between 20 and 65%, relative to the starting length of the implant.

According to a preferred embodiment, the implant is made from at least one protein, in particular one protein. The at least one protein is preferably a natural protein, in particular a xenogenic protein, preferably bovine, porcine and/or equine protein. A protein of bovine origin is particularly preferred. In a further embodiment, the at least one protein is an extracellular protein, preferably a fibrous extracellular protein. The at least one protein is particularly preferably chosen from the group comprising collagen, gelatin, reticulin, elastin and combinations thereof. Collagen is particularly preferred according to the invention. Suitable collagens are in particular fibrillar collagens, preferably collagens of type I, II, III, V and/or VI. The collagens can also be tendon collagen and/or ligament collagen.

In one possible embodiment, the implant is made from at least one protein of recombinant origin. For example, such a protein can be produced by microorganisms, in particular yeast cells. For further features and properties of the at least one recombinant protein, reference is made to the preceding description.

In another embodiment, the implant is sponge-like. The implant is preferably present as a non-woven fabric, a fibrous web or a card web, in particular a fleece. Preferably, the implant is present as a sprayed non-woven fabric, sprayed fibrous web, sprayed card web or as a meltblown fabric, in particular a spray fleece. The implant can in principle have a two-layer, three-layer or multi-layer structure. However, the implant is preferably formed in one layer.

The implant also preferably has a thickness of 1 to 10 mm, in particular of > 4 mm, preferably of 6 to 8 mm. The implant can also have a weight per unit area of between 30 and 300 g/m², in particular of between 80 and 120 g/m².

In another embodiment, the implant has an absorption capacity for liquids, in particular for body fluids, preferably blood, corresponding to 10 to 60 times, in particular 25 to 40 times its own weight when dry. In particular, the implant can be wetted with water completely, i.e. on its outer and inner surfaces, within a period of < 100 seconds, in particular of < 60 seconds. As regards blood, the implant can particularly advantageously be wetted within a period of < 180 seconds.

To further increase the liquid absorption capacity, the implant can comprise a biocompatible organic acid. Examples of suitable acids in this respect are acids from the group comprising citric acid, tartaric acid, ascorbic acid, malic acid, gluconic acid, mucic acid, glutaric acid, adipic acid and combinations thereof. By the addition of an acid, the implant can have a pH value of < 4 upon contact with water.

The implant according to the invention can also comprise active substances, for example antimicrobial, antiseptic, anti-inflammatory, growth-promoting and/or deodorizing active substances. Examples of antimicrobial active substances are antibiotics, in particular gentamicin and/or rifampicin. Preferred antimicrobial active substances are antimicrobial metals and/or antimicrobial metal compounds, preferably antimicrobial metal salts, in particular silver salts, for example silver acetate. Examples of suitable anti-inflammatory active substances can be chosen from the group comprising allatonin, saponin, riboflavin, flavonoids, tocopherol, β-sitosterol, soledum cineol, dexpanthenol, bromalain and combinations thereof. In principle, active substances contained in the implant can be present in particle form, in particular as nanoparticles and/or microparticles.

According to the invention, it can also be advantageous if the implant is at least partially coloured, preferably completely coloured. The implant can generally be coloured with the aid of a dye suitable for this purpose, for example from the group comprising D&C dyes (drug and cosmetic dyes), riboflavin, retinol, methylene blue and combinations thereof. By colouring the implant, it is possible to avoid a situation in which an otherwise white implant dazzles the surgeon during a surgical procedure. For example, the implant can be coloured yellow by riboflavin.

In another preferred embodiment, the implant is present as lyophilisate. Increased dimensional stability of the implant is achieved in a particularly advantageous manner by lyophilization.

The implant is generally present in sterilized form. The sterilization methods that can be used are in principle all methods known to a person skilled in the art. For example, the implant according to the invention can be present in sterilized form following γ-sterilization, ethylene oxide gas sterilization or plasma sterilization. Moreover, the implant can be present in a packaged form. To this extent, the depressions provided according to the invention on the implant surface can facilitate preliminary packaging.

As has already been mentioned at the outset, the implant according to the invention is suitable in particular for sealing or closing liquid and/or air leaks in the human and/or animal body. The implant according to the invention is preferably used as a haemostatic or haemostyptic. In other words, the implant is preferably designed as a haemostyptic or haemostatic. In this form, the implant can be used for haemostatic treatment of internal and external wounds. The implant is preferably suitable for haemostasis of internal wounds, in particular for haemorrhages of parenchymatous organs, for example the liver, spleen, pancreas, kidneys, adrenal glands or lungs. Organ haemorrhages of this kind may occur especially after trauma or (partial) resections. The implant is also suitable for sealing or closing leaks of cerebrospinal fluid or as a replacement for the dura mater or as an onlay for the dura mater. Another possible area of use is the closure of anastomoses, in particular vascular or intestinal anastomoses. Moreover, the implant according to the invention is also suitable for sealing leaks of the bladder, ureter, lungs and/or pericardium.

A further aspect of the present invention concerns a method for the production of the implant according to the invention, wherein predetermined bending locations in the form of depressions are formed on the surface of a planar implant.

The implant is preferably lyophilized before the depressions are formed. In one possible embodiment, the depressions are formed on the implant surface during a lyophilization step. This can generally be done using suitable structured models of lattices and/or nets, but also using rods or profiled (structured) lyophilization dishes.

According to a particularly preferred embodiment, the depressions are formed on the surface of the implant by being forced in, in particular by pressing, preferably embossing. According to the invention, it is particularly preferable if the depressions are formed using pressing dies, preferably embossing dies, in particular in the form of profiled embossing plates, profiled embossing rollers, nets, lattices or patterned templates. For production of profiled embossing plates, plates without profiling can be subjected to a milling, etching or laser procedure or cast to a final shape.

To form the depressions on the implant surface, the implant is preferably placed in a suitable press. Suitable presses can be operated pneumatically, hydraulically or electrically. Examples of suitable presses within the meaning of the invention are swivel arm presses, column presses, die presses, calender roller presses and calender presses with embossing patterns. If appropriate, the implant can be placed for a pressing operation onto a suitable substrate, for example a rubber support.

Further features and advantages of the invention will become clear from the following description of the figures and from the following example in conjunction with the features of the dependent claims. Individual features can be realized either singly or several of them in combination.

In the figures:
Figure 1 shows a schematic side view of an embodiment of the implant according to the invention,
Figure 2 shows a schematic plan view of various embodiments of the implant according to the invention,
Figure 3 shows the compression strength [N] of implants according to the invention compared to a conventional implant,
Figure 4 shows the tear strength [N] of implants according to the invention compared to a conventional implant,
Figure 5 shows the elongation at break [%] of implants according to the invention compared to a conventional implant.

### Description of the figures

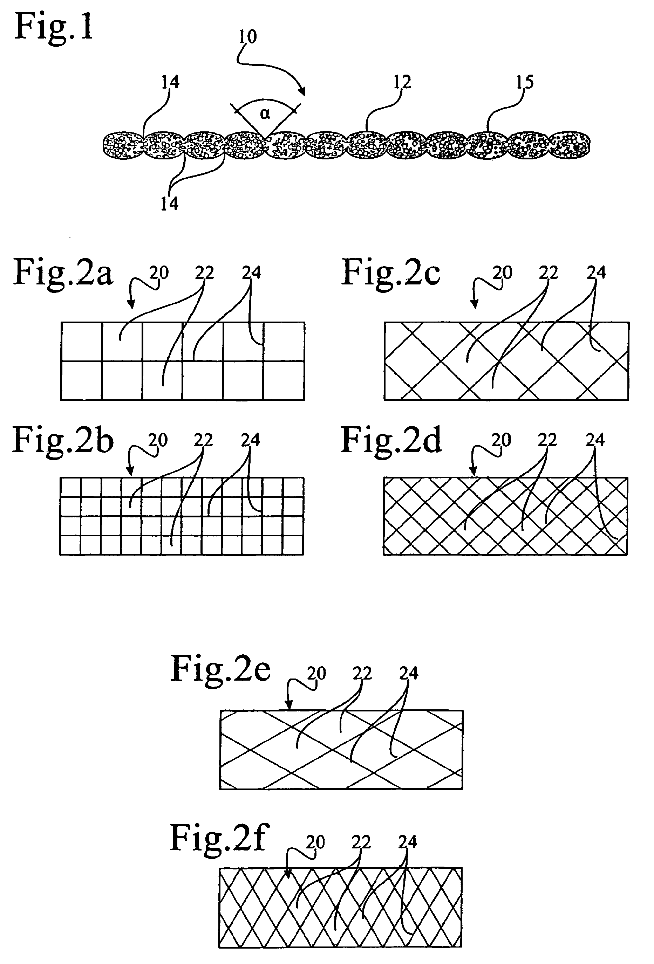

Figure 1 shows a schematic side view of an implant 10 according to the invention. On both sides of its surface 12, the implant 10 has depressions 14 dividing the surface 12 into a multiplicity of elevations 15. The elevations 15 can be completely arched or have at least one plateau. The surface 12 drops down to the depressions 14 at an angle α, as a result of which no tensile stress occurs, for example when the implant 10 is bent. In this way, the risk of the implant breaking can be greatly reduced.

Figure 2 shows a schematic plan view of various embodiments of an implant 20 according to the invention. The depressions 24 are formed in a linear configuration in the form of a pattern on the surface 22 of the implant 20. In Figures 2a and 2b, depressions 24 are provided in the form of a pattern of squares on the surface 22 of the implant 20. By contrast, in Figures 2c-2f, the depressions 24 are provided on the implant surface 22 in the form of a pattern of diamonds or lozenges. The patterns in Figures 2a-f each differ from one another in terms of their grid size.

Taking the example of collagen fleeces with an embossed pattern, Figure 3 shows in graph form the compression strength, measured in newtons (ordinate), of implants according to the invention (abscissa: numbers 2 to 11) compared to a collagen fleece without an embossed pattern and known from the prior art (abscissa: number 1). As examples of implants according to the invention, the following were tested: collagen fleeces with a 1 x 1 cm embossed pattern of squares (abscissa: number 2), a 1.5 x 1.5 cm embossed pattern of squares (abscissa: number 3), a 2 x 2 cm embossed pattern of squares (abscissa: number 4), a 1 x 1 cm embossed pattern of lozenges (abscissa: number 5), a 1.5 x 1.5 cm embossed pattern of lozenges (abscissa: number 6), a 2 x 2 cm embossed pattern of lozenges (abscissa: number 7), a 0.8 x 1.5 cm embossed pattern of diamonds (abscissa: number 8), a 1.0 x 2.5 cm embossed pattern of diamonds (abscissa: number 9), a 1.5 x 3.5 cm embossed pattern of diamonds (abscissa: number 10), and a 2.5 x 3.5 cm embossed pattern of diamonds (abscissa: number 11).

To determine the compression strength, the fleeces were each cut to a size of 25 x 100 mm. The free clamped length was 70 mm. At a test speed of 50 mm/min, the fleeces were each compressed to 35 mm (corresponding to 50% of the starting length). The maximum force having to be applied for compression of the fleeces was detected. The results depicted in graph form in Figure 3 show that the compression strength measured using the collagen fleeces with an embossed pattern is significantly less than for the collagen fleece without embossing and serving as a reference value. This confirms the extraordinarily high degree of flexibility of fleeces according to the invention compared to conventional collagen fleeces.

Taking the example of collagen fleeces with an embossed pattern, Figure 4 shows in graph form the tear strength, measured in newtons (ordinate), of implants according to the invention (abscissa: numbers 2 to 11) compared to a collagen fleece without an embossed pattern and known from the prior art (abscissa: number 1). As examples of implants according to the invention, the following were tested: collagen fleeces with a 1 x 1 cm embossed pattern of squares (abscissa: number 2), a 1.5 x 1.5 cm embossed pattern of squares (abscissa: number 3), a 2 x 2 cm embossed pattern of squares (abscissa: number 4), a 1 x 1 cm embossed pattern of lozenges (abscissa: number 5), a 1.5 x 1.5 cm embossed pattern of lozenges (abscissa: number 6), a 2 x 2 cm embossed pattern of lozenges (abscissa: number 7), a 0.8 x 1.5 cm embossed pattern of diamonds (abscissa: number 8), a 1.0 x 2.5 cm embossed pattern of diamonds (abscissa: number 9), a 1.5 x 3.5 cm embossed pattern of diamonds (abscissa: number 10), and a 2.5 x 3.5 cm embossed pattern of diamonds (abscissa: number 11).

To determine the tear strength, the fleeces were each cut to a size of 25 x 100 mm. The free clamped length chosen was 70 mm. The fleeces were then pulled at a test speed of 30 mm/min until the fleeces broke. The maximum force that had to be applied for this was detected. The results depicted in graph form in Figure 4 show that, in the collagen fleeces with embossed patterns, a significantly increased tear strength was measured by comparison to the collagen fleece without embossing. This underlines the extraordinary stability of the collagen fleeces with an embossed pattern.

Taking the example of collagen fleeces with an embossed pattern, Figure 5 shows in graph form the elongation at break, in percentages (ordinate), of implants according to the invention (abscissa: numbers 2 to 11) compared to a collagen fleece without an embossed pattern and known from the prior art (abscissa: number 1). As examples of implants according to the invention, the following were tested: collagen fleeces with a 1 x 1 cm embossed pattern of squares (abscissa: number 2), a 1.5 x 1.5 cm embossed pattern of squares (abscissa: number 3), a 2 x 2 cm embossed pattern of squares (abscissa: number 4), a 1 x 1 cm embossed pattern of lozenges (abscissa: number 5), a 1.5 x 1.5 cm embossed pattern of lozenges (abscissa: number 6), a 2 x 2 cm embossed pattern of lozenges (abscissa: number 7), a 0.8 x 1.5 cm embossed pattern of diamonds (abscissa: number 8), a 1.0 x 2.5 cm embossed pattern of diamonds (abscissa: number 9), a 1.5 x 3.5 cm embossed pattern of diamonds (abscissa: number 10), and a 2.5 x 3.5 cm embossed pattern of diamonds (abscissa: number 11).

To determine the elongation at break, the fleeces were cut to a size of 25 x 100 mm and clamped by a length of 70 mm. The fleeces were each pulled at a test speed of 30 mm/min until the fleeces broke, and the maximum elongation (in %) at maximum force was detected. The results depicted in graph form in Figure 5 show that, in the embossed collagen fleeces, the elongation at break is predominantly greater than in the collagen fleece without embossing and serving as reference value.

In summary, the values measured by way of example on the basis of collagen fleeces and pertaining to compression strength (Figure 3), tear strength (Figure 4) and elongation at break (Figure 5) confirm that implants according to the invention are distinguished from conventional implants by having greater flexibility, in particular bendability, and increased stability.

### Example:

### Production of an implant according to the invention

66 g of collagen were swollen in 1,320 ml of ultrapure water (MilliQ-Wasser, from the Millipore company, Germany). The swollen collagen was then suspended for ten minutes in a solvent mixture composed of 2,310 ml of ultrapure water and 330 ml of isopropanol. Thereafter, 520 g of the suspension were in each case poured onto lyophilization dishes with a bottom surface area of 660 cm² and a height of 3 cm, frozen at -30°C and then lyophilized. Rectangular collagen plates were obtained as the product. The thickness of the plates was dependent on the filling level of the dishes. The layer thickness of the suspension filling was critical for the end thickness of the collagen plates. The lyophilized collagen plates were embossed by means of a structured calender roller, with formation of grid-shaped depressions on the surface. The collagen plates, in a grid of 15 mm, were embossed diagonally at a 45° angle and 1 mm web width, to a residual thickness of 0.8 mm. After relaxation, the embossings had a thickness of 20 to 40% of the non-embossed areas of the fleece.

## Claims

1. Planar implant (10; 20) for sealing liquid and/or air leaks in the human and/or animal body, wherein the implant (10; 20) has a surface (12; 22) with predetermined bending locations in the form of depressions (14; 24), and wherein the implant (10; 20) is composed of at least one protein **characterized in that** the implant (10; 20) is compacted in the area of the depressions (14; 24) and the depressions (14; 24) are formed in a lattice shape or net shape on the surface (12; 22) of the implant (10; 20).

2. Planar implant (10; 20) according to Claim 1, **characterized in that** the depressions (14; 24) are elongate, preferably linear.

3. Planar implant (10; 20) according to Claim 1 or 2, **characterized in that** the depressions (14; 24) are groove-shaped or channel-shaped.

4. Planar implant (10; 20) according to one of the preceding claims, **characterized in that** the depressions (14; 24) are continuous depressions.

5. Planar implant (10; 20) according to one of the preceding claims, **characterized in that** the implant (10; 20) is pressed, preferably embossed, in the area of the depressions (14; 24).

6. Planar implant (10; 20) according to one of the preceding claims, **characterized in that** the depressions (14; 24) are pressed depressions, in particular punched or embossed depressions.

7. Planar implant (10; 20) according to one of the preceding claims, **characterized in that** the depressions (14; 24) are formed on the implant surface (12; 22) in a grid size of between 0.5 and 3 cm.

8. Planar implant (10; 20) according to one of the preceding claims, **characterized in that** the depressions (14; 24) are formed on the surface (12; 22) of the implant (10; 20) in a polygonal pattern, in particular in a pattern of triangles, diamonds, lozenges, squares, rectangles, pentagons or hexagons.

9. Planar implant (10; 20) according to one of the preceding claims, **characterized in that** the depressions (14; 24) are formed on at least one side face, preferably on two side faces, of the implant (10; 20).

10. Planar implant (10; 20) according to one of the preceding claims, **characterized in that** the implant (10; 20), in the area of the depressions (14; 24), has a thickness of between 1 and 90%, preferably of between 20 and 80%, particularly preferably of between 60 and 80%, relative to an implant cross section without depressions.

11. Planar implant (10; 20) according to one of the preceding claims, **characterized in that** the implant (10; 20) is composed of at least one extracellular protein, particularly preferably of collagen.

12. Planar implant (10; 20) according to one of the preceding claims, **characterized in that** the implant (10; 20) is present as a non-woven fabric, in particular a fleece, preferably a spray fleece.

13. Planar implant (10; 20) according to one of the preceding claims, **characterized in that** the implant (10; 20) is present as lyophilisate.

14. Planar implant (10; 20) according to one of the preceding claims, for haemostasis of internal wounds, in particular of haemorrhages of parenchymatous organs, for sealing leaks of cerebrospinal fluid, as replacement material for the dura mater or for sealing leaks of the bladder, ureter, lungs and/or pericardium.

15. Method for the production of an implant (10; 20) according to one of the preceding claims, wherein predetermined bending locations in the form of depressions (14; 24) are formed on the surface (12; 22) of a planar implant.

16. Method according to claim 15, **characterized in that** the implant is lyophilized before the depressions (14; 24) are formed.

## Patentansprüche

1. Planares Implantat (10; 20) zur Abdichtung von Flüssigkeits- und/oder Luftlecks im menschlichen und/oder tierischen Körper, wobei das Implantat (10; 20) eine Oberfläche (12; 22) mit vorbestimmten Biegestellen in Form von Vertiefungen (14; 24) aufweist und wobei das Implantat (10; 20) aus mindestens einem Protein gebildet ist, **dadurch gekennzeichnet, dass** das Implantat (10; 20) im Bereich der Vertiefungen (14; 24) verdichtet ist und die Vertiefungen (14; 24) in einer Gitterform oder Netzform auf der Oberfläche (12; 22) des Implantats (10; 20) ausgebildet sind.

2. Planares Implantat (10; 20) nach Anspruch 1, **dadurch gekennzeichnet, dass** die Vertiefungen (14; 24) langgestreckt, bevorzugt linear, ausgebildet sind.

3. Planares Implantat (10; 20) nach Anspruch 1 oder 2, **dadurch gekennzeichnet, dass** die Vertiefungen (14; 24) rinnenförmig oder kanalförmig ausgebildet sind.

4. Planares Implantat (10; 20) nach einem der vorhergehenden Ansprüche, **dadurch gekennzeichnet, dass** die Vertiefungen (14; 24) als durchgehende Vertiefungen ausgebildet sind.

5. Planares Implantat (10; 20) nach einem der vorhergehenden Ansprüche, **dadurch gekennzeichnet, dass** das Implantat (10; 20) im Bereich der Vertiefungen (14; 24) mit Einpressungen, bevorzugt mit Prägungen, ausgebildet ist.

6. Planares Implantat (10; 20) nach einem der vorhergehenden Ansprüche, **dadurch gekennzeichnet, dass** die Vertiefungen (14; 24) als eingepresste Vertiefungen, insbesondere als geschlagene oder geprägte Vertiefungen, ausgebildet sind.

7. Planares Implantat (10; 20) nach einem der vorhergehenden Ansprüche, **dadurch gekennzeichnet, dass** die Vertiefungen (14; 24) auf der Implantatoberfläche (12; 22) mit einer Rasterabmessung zwischen 0,5 und 3 cm ausgebildet sind.

8. Planares Implantat (10; 20) nach einem der vorhergehenden Ansprüche, **dadurch gekennzeichnet, dass** die Vertiefungen (14; 24) auf der Oberfläche (12; 22) des Implantats (10; 20) in einem polygonalen Muster, insbesondere in einem Muster von Dreiecken, Rauten, Rhomben, Quadraten, Rechtecken, Fünfecken oder Sechsecken ausgebildet sind.

9. Planares Implantat (10; 20) nach einem der vorhergehenden Ansprüche, **dadurch gekennzeichnet, dass** die Vertiefungen (14; 24) auf mindestens einer Seitenfläche, bevorzugt auf zwei Seitenflächen, des Implantats (10; 20) ausgebildet sind.

10. Planares Implantat (10; 20) nach einem der vorhergehenden Ansprüche, **dadurch gekennzeichnet, dass** das Implantat (10; 20), im Bereich der Vertiefungen (14; 24), eine Dicke zwischen 1 und 90 %, bevorzugt zwischen 20 und 80 %, besonders bevorzugt zwischen 60 und 80 %, bezogen auf einen Implantatquerschnitt ohne Vertiefungen aufweist.

11. Planares Implantat (10; 20) nach einem der vorhergehenden Ansprüche, **dadurch gekennzeichnet, dass** das Implantat (10; 20) aus mindestens einem extrazellulären Protein, besonders bevorzugt aus Kollagen, gebildet ist.

12. Planares Implantat (10; 20) nach einem der vorhergehenden Ansprüche, **dadurch gekennzeichnet, dass** das Implantat (10; 20) als Flächengebilde aus Faserstoff (Nonwoven), insbesondere als Vlies, bevorzugt als Sprühvlies, vorliegt.

13. Planares Implantat (10; 20) nach einem der vorhergehenden Ansprüche, **dadurch gekennzeichnet, dass** das Implantat (10; 20) als Lyophilisat vorliegt.

14. Planares Implantat (10; 20) nach einem der vorhergehenden Ansprüche, zur Hämostase bei inneren Wunden, insbesondere bei Hämorrhagien von Parenchymorganen, zum Abdichten von Leckagen von Zerebrospinalflüssigkeit, als Ersatzmaterial für die Dura Mater oder zum Abdichten von Leckagen an Blase, Ureter, Lungen und/oder Perikard.

15. Verfahren zur Herstellung eines Implantats (10; 20) nach einem der vorhergehenden Ansprüche, bei dem vorbestimmte Biegestellen in Form von Vertiefungen (14; 24) auf der Oberfläche (12; 22) eines planaren Implantats ausgebildet werden.

16. Verfahren nach Anspruch 15, **dadurch gekennzeichnet, dass** das Implantat lyophilisiert wird, bevor die Vertiefungen (14; 24) ausgebildet werden.

## Revendications

1. Implant plan (10 ; 20) pour étanchéifier des fuites de liquide et/ou d'air dans le corps humain et/ou animal, l'implant (10 ; 20) ayant une surface (12 ; 22) avec des emplacements de flexion prédéterminés sous la forme de dépressions (14 ; 24), et l'implant (10 ; 20) étant constitué d'au moins une protéine, **caractérisé en ce que** l'implant (10 ; 20) est compacté dans la zone des dépressions (14 ; 24) et les dépressions (14 ; 24) sont formées en forme de treillis ou de filets sur la surface (12 ; 22) de l'implant (10 ; 20).

2. Implant plan (10 ; 20) selon la revendication 1, **caractérisé en ce que** les dépressions (14 ; 24) sont allongées, de préférence linéaires.

3. Implant plan (10 ; 20) selon la revendication 1 ou 2, **caractérisé en ce que** les dépressions (14 ; 24) sont en forme de gorges ou de canaux.

4. Implant plan (10 ; 20) selon l'une quelconque des revendications précédentes, **caractérisé en ce que** les dépressions (14 ; 24) sont des dépressions continues.

5. Implant plan (10 ; 20) selon l'une quelconque des revendications précédentes, **caractérisé en ce que** l'implant (10 ; 20) est pressé, de préférence gaufré dans la zone des dépressions (14 ; 24).

6. Implant plan (10 ; 20) selon l'une quelconque des revendications précédentes, **caractérisé en ce que** les dépressions (14 ; 24) sont des dépressions pressées, en particulier des dépressions poinçonnées ou gaufrées.

7. Implant plan (10 ; 20) selon l'une quelconque des revendications précédentes, **caractérisé en ce que** les dépressions (14 ; 24) sont formées sur la surface (12 ; 22) de l'implant dans une dimension de grille comprise entre 0,5 et 3 cm.

8. Implant plan (10 ; 20) selon l'une quelconque des revendications précédentes, **caractérisé en ce que** les dépressions (14 ; 24) sont formées sur la surface (12 ; 22) de l'implant (10 ; 20) suivant un motif polygonal, en particulier un motif en triangles, en diamants, en losanges, en carrés, en rectangles, en pentagones ou en hexagones.

9. Implant plan (10 ; 20) selon l'une quelconque des revendications précédentes, **caractérisé en ce que** les dépressions (14 ; 24) sont formées sur au moins une face latérale, de préférence sur deux faces latérales, de l'implant (10 ; 20).

10. Implant plan (10 ; 20) selon l'une quelconque des revendications précédentes, **caractérisé en ce que** l'implant (10 ; 20), dans la zone des dépressions (14 ; 24), présente une épaisseur comprise entre 1 et 90 %, de préférence entre 20 et 80 %, particulièrement préférablement entre 60 et 80 %, par rapport à une section transversale d'implant sans dépressions.

11. Implant plan (10 ; 20) selon l'une quelconque des revendications précédentes, **caractérisé en ce que** l'implant (10 ; 20) est constitué d'au moins une protéine extracellulaire, en particulier de préférence de collagène.

12. Implant plan (10 ; 20) selon l'une quelconque des revendications précédentes, **caractérisé en ce que** l'implant (10 ; 20) est présent sous forme de tissu non-tissé, en particulier une nappe non tissée, de préférence un spray tissu non-tissé.

13. Implant plan (10 ; 20) selon l'une quelconque des revendications précédentes, **caractérisé en ce que** l'implant (10 ; 20) est présent sous forme de lyophilisat.

14. Implant plan (10 ; 20) selon l'une quelconque des revendications précédentes, pour l'hémostase de plaies internes, en particulier d'hémorragies d'organes parenchymateux, pour étanchéifier des fuites de fluide cérébrospinal, en tant que matériau de remplacement pour la dure-mère ou pour étanchéifier des fuites dans la vessie, l'urètre, les poumons et/ou le péricarde.

15. Procédé de production d'un implant (10 ; 20) selon l'une quelconque des revendications précédentes, dans lequel des emplacements de flexion prédéterminés sous la forme de dépressions (14 ; 24) sont formés sur la surface (12 ; 22) d'un implant plan.

16. Procédé selon la revendication 15, **caractérisé en ce que** l'implant est lyophilisé avant que les dépressions (14 ; 24) ne soient formées.
